# EUROPEAN PATENT APPLICATION

(11) **EP 1 510 230 A1**
(43) Date of publication of application: **02.03.2005**
(21) Application number: 04077727.8
(22) Date of filing: 18.02.2000
(51) Int. Cl.: A61M 5/142

(54) **Negative pressure medical infusion pump**

(30) Priority: 19.02.1999 US 253383
(62) Divisional of application: 00908735.4
(71) Applicant: Medical Research Group, Inc., Sylmar, CA 91342-3728 (US); MiniMed Inc., Sylmar, California 91342-3728 (US)
(72) Inventor: The designation of the inventor has not yet been filed
(74) Representative: Reeve, Anna Elizabeth

(57) **Abstract**

A medication infusion pump (10; 110) comprises a housing (12;112), a medication chamber (14;114) defined within the housing (12;112), a pumping mechanism for supplying (16;116) the medication from the medication chamber (14;114) to a patient, a reservoir (28;122) defined within the housing (12;112) and adapted to apply a predetermined pressure less than ambient atmospheric pressure to the medication chamber (14;114), and a gas within the reservoir (28;122). The gas has a vapor pressure less than ambient atmospheric pressure at normal human physiological temperature. The gas is selected from the group consisting of cyclopentane, 2,2-dimethylbutane, 1,3-pentadiene, and 2-methyl-2-butene and does not have an adverse effect on ozone.

## Description

### FIELD OF THE INVENTION

The present invention relates to medication infusion pumps, more particularly implantable infusion pumps.

### BACKGROUND OF THE INVENTION

Medication infusion pumps that supply a medication to a patient, in particular implantable infusion pumps, are known. Known infusion pumps typically include a housing that contains a medication chamber and a propellant chamber, together with appropriate means for loading the selected medication into the medication chamber and for supplying the medication to the patient. One known type of infusion pump includes a reservoir containing a gas at a pressure below atmospheric pressure. Such infusion pumps are characterized as "negative pressure" pumps. Negative pressure pumps offer the advantage of facilitating fill of the medication chamber by acting to draw in the medication from a hypodermic needle or other supply source into the medication chamber. Such pumps also have the advantage of inhibiting undesirable leakage from the medication chamber.

Negative pressure infusion pumps are described, for example, in U.S. Patent No. 4,191,181 (France Pat. Pub. No. 2,370,481, Germany Pat. Pub. No. 2652026, and Great Britain Pat. Pub. No. 1,554,083), to Frenetzki et al.; U.S. Patent No. 4,373,527 (France Pat. Pub. No. 2,537,873, Germany Pat. Pub. No. 3247232, and Great Britain Pat. Pub. No. 2,132,090), to Fischell et al.; U.S. Patent No. 4,511,355 (European Pat Pub. No. 75,762), to Frenetzki et al.; U.S. Patent No. 4,573,994 (European Pat Pub. No. 95,506), to Fischell et al.; and U.S. Patent No. 5,514,103 (European Pat Pub. No. 687,475), to Srisathapat et al. Each of these patent publications is hereby incorporated herein by reference as if set forth herein. In a typical negative pressure infusion pump, a medication is drawn into the medication chamber under the action of a gas maintained in a flexible reservoir at negative pressure. Once the medication is drawn into the medication chamber, the medication is subsequently delivered to the patient via a separate pumping mechanism.

Modern environmental concerns mandate that gases employed in "negative pressure" pumps be environmentally benign. It is of particular concern to avoid gases that have an adverse effect on ozone.

A need exists for a negative pressure infusion pump that employs an environmentally acceptable gas in its gas reservoir.

### SUMMARY OF THE PREFERRED EMBODIMENTS

A first aspect of the invention provides a medication infusion pump comprising
a) a housing,
b) a medication chamber defined within the housing,
c) means for supplying the medication from the medication chamber to a patient,
d) a reservoir defined within the housing and adapted to apply a predetermined pressure less than ambient atmospheric pressure to the medication chamber, and
e) a gas within the reservoir, the gas having a vapor pressure less than ambient atmospheric pressure at normal human physiological temperature, characterised in that the gas is selected from the group consisting of cyclopentane, 2,2-dimethylbutane, 1,3-pentadiene, and 2-methyl-2-butene and does not have an adverse effect on ozone.

Preferably, the medication infusion pump is adapted to be passively refilled with a medication.

Preferably, the gas comprises a non-toxic gas.

In a preferred embodiment the gas has a vapor pressure less than 14.7 psia (101 kPa) at 37°C.

Preferably, the gas has a vapor pressure less than 10.7 psia (74 kPa) at 37°C.

In a preferred embodiment, the gas is selected from the group consisting of cyclopentane and dimethylbutane.

Preferably, the medication infusion pump comprises a plurality of gases within the reservoir, wherein the plurality of gases has a combined vapor pressure less than 14.7 psia (101 kPa) at 37°C.

Preferably, the medication chamber and the reservoir are separated by a barrier which is resistant to gas.

A second aspect of the invention provides a medication infusion pump for use in a method of supplying a medication to a patient from a medication infusion pump, the infusion pump including a housing, a medication chamber defined within the housing, a pumping mechanism for supplying the medication from the medication chamber to the patient, and a reservoir defined within the housing and adapted to apply a predetermined pressure less than ambient atmospheric pressure to the medication chamber, the method comprising the steps of
(i) filling the reservoir with a gas having a vapor pressure less than ambient atmospheric pressure at normal human physiological temperature, the gas being selected from the group consisting of cyclopentane, 2,2-dimethylbutane, 1,3-pentadiene, and 2-methyl-2-butene, and wherein the gas does not have an adverse effect on ozone,
ii) drawing the medication into the medication chamber by exertion of the predetermined negative pressure on the medication, and
iii) dispensing the medication from the medication chamber via the pumping mechanism.

Other objects, features and advantages of the present invention will become apparent to those skilled in the art from the following detailed description. It is to be understood, however, that the detailed description and specific examples, while indicating preferred embodiments of the present invention, are given by way of illustration and not limitation.

### Brief Description of the Drawings

The invention may be more readily understood by referring to the accompanying drawings in which
Fig. 1 is a side sectional view of a negative pressure medication infusion pump according to the present invention that employs a flexible bladder as the gas reservoir, and
Fig. 2 is a schematic representation, partially cut-away, of a portion of an alternative embodiment of a negative pressure medication infusion pump according to the present invention that employs a corrugated gas reservoir.

### DETAILED DESCRIPTION OF THE PREFERRED EMBODIMENTS

Gases are "non-toxic" for the purposes of this invention if they comply with the standard established in ISO 10993. Gases are also "non-toxic" for the purposes of this invention if they are included in the inventory of the United States Toxic Substances Control Act.

A gas is considered "non-ozone depleting" for the purposes of this invention if (i) the gas has an Ozone Depletion Potential (ODP) less than 1.0, more preferably less than about 0.10 (with the ODP of CFC-11=1.0) and either (ii) the gas is not a Class I or Class II controlled substance as set forth in Volume 40 of the United States Code of Federal Regulation Part 82, Subpart A (40 C.F.R. Part 82, Subpart A), or (iii) the gas is subject to the exemption provided for under 40 C.F.R. Part 82.4(s)(1) (use in a medical device). Determination of the ODP of a gas is readily carried out by those skilled in the art.

Gases useful according to the present invention have a vapor pressure less than ambient atmospheric pressure at normal human physiological temperature (37°C). More particularly, the gas has a vapor pressure less than sea level atmospheric pressure (i.e. 14.7 psia (101 Pa) at 37°C. Preferably, the gas has a vapor pressure less than 10.7 psia (74 kPa) at 37°C. Exemplary gases include cyclopentane (vapor pressure 9.4 psia (65 kPa) at 37°C) and 2,2-dimethylbutane (vapor pressure 9.6 psia (66 kPa) at 37°C). Other useful gases include 1,3-pentadiene (vapor pressure 12.50 psia (86 kPa) at 37°C), 2-methyl-2-butene (vapor pressure 14.24 psia (98 kPa) at 37°C), and methyl iodide (vapor pressure 11.10 psia (77 kPa) at 37°C). Other branched chain fluoroalkanes and fluoroalkenes are also suitable for use according to the instant invention.

Gases having vapor pressures lower than 10.7 psia (74 kPa) at 37°C are particularly desirable in that infusion pumps that employ such gases can be effectively used even at relatively high altitudes. For example, the vapor pressure of 2,2-dimethylbutane, 9.4 psia (65 kPa) corresponds to an altitude of about 12,000 feet (3.7 kilometres) above sea level. The vapor pressure of cyclopentane, 9.6 psia (66 kPa) corresponds to an altitude of about 11,500 feet (3.5 kilometres). Thus, use of the foregoing preferred gases in accordance with the present invention allows the infusion pump to be employed in almost all major population centers and most mountain areas, as well as in aircraft. When implanted in a human patient, these embodiments of the infusion pump allow the patient to travel freely.

It is also contemplated that mixture of two or more gases are also useful according to the instant invention, provided that the mixture is capable of maintaining a pressure less than 14.7 psia (101 kPa), more preferably less than 10.7 psia (74 kPa).

Desirably, the gas or gas mixture maintains the reservoir at a pressure less than 14.7psia (101 kPa), more preferably less than 10.7psia (74kPa), at 37°C when the medication chamber is filled and also when the medication is unfilled. The stability of the vapor pressure may be obtained by ensuring that a sufficient quantity of the fill gas material (i.e. liquid phase and gas phase material) is placed in the fill gas reservoir. This may be achieved by ensuring that some liquid phase portion of the fill gas material remains in the reservoir when the medication chamber is empty. It is also preferred that some of the fill gas remain in the gaseous state when the medication chamber is full.

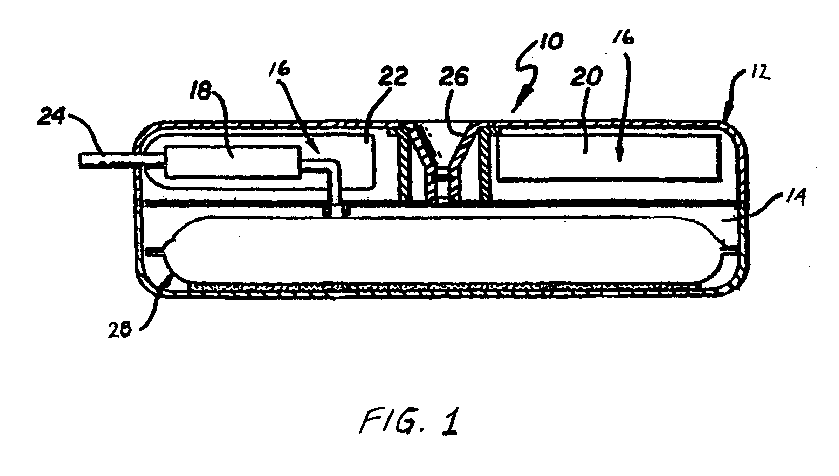

Turning now to Figure 1, a schematic representation of a first embodiment of a medication infusion pump 10 according to the invention includes a housing 12 within which a medication chamber 14 is defined. A pumping mechanism 16 is provided for supplying a medication from medication chamber 14 to a patient. The pumping mechanism 16 includes a pump 18, control circuitry 20, battery 22 and catheter 24. Other known means for supplying a medication from medication chamber 14 can also be employed.

A medication is loaded into medication chamber 14, for example, via an inlet or refill fitting 26 adapted to receive a hypodermic needle (not shown).

Reservoir 28 is defined within housing 12. In the illustrated embodiment, reservoir 28 is a flexible and expansible bag formed from a polymeric material and secured within housing 12. Alternatively, reservoir 28 can be defined by a flexible membrane that extends across the interior of housing 12. The selected gas or gas mixture is supplied to reservoir 28 at the selected pressure by conventional means known to those skilled in the art.

A medication is loaded into medication chamber 14, for example, via an inlet or refill fitting 26 adapted to receive a hypodermic needle (not shown). Due to the negative pressure maintained in reservoir 28, the medication is passively drawn into the medication chamber 14, and is subsequently dispensed via pump 18.

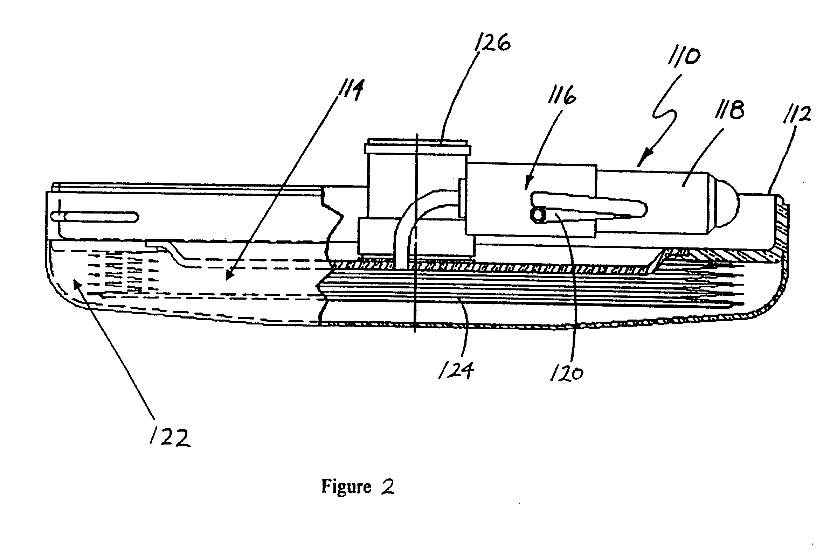

In Figure 2, an alternative embodiment of a medication infusion pump 110 according to the invention employs a reservoir defined by a flexible corrugated structure. Housing 112 has defined therein medication chamber 114. Pumping mechanism 116 for supplying the medication from medication chamber 114 to a patient includes pump 118, control circuitry and battery (not shown), and catheter 120.

Reservoir 122 is defined within housing 112 and is separated from medication chamber 114 by flexible corrugated structure 124. Structure 124 is preferably formed from a corrugated metal, for example titanium. The material used to form structure 124 is preferably substantially impermeable to the gas mixture, the selected medication, and any gases dissolved in the medication. The selected gas or gas mixture is supplied to reservoir 122 by conventional means known to those skilled in the art.

While particular preferred embodiments of the present invention have been described above, it will be understood by those of skill in the art that many modifications and variations of the teachings may be made.

In a preferred embodiment preferably a medication infusion pump comprises:
a) a housing,
b) a medication chamber defined within the housing,
c) means for supplying the medication from the medication chamber to a patient,
d) a reservoir defined within the housing and adapted to apply a predetermined negative pressure to the medication chamber, and
e) a gas within the reservoir, the gas having a vapor pressure less than ambient atmospheric pressure at normal human physiological temperature.

Preferably, the medication infusion pump is adapted to be passively refilled with a medication, the medication infusion pump comprising a reservoir that contains a gas having a vapor pressure less than ambient atmospheric pressure at normal human physiological temperature.

Preferably, the gas comprises a non-ozone depleting gas.

Preferably, the gas comprises a non-toxic gas.

Preferably, the gas has a vapor pressure less than 14.7 psia at 37°C.

Preferably, the gas has a vapor pressure less than 10.7 psia at 37°C.

Preferably, the gas is selected from the group consisting of cyclopentane, 2,2-dimethylbutane, 1 ,3-pentadiene and 2-methyl-2-butene.

Preferably, the gas is selected from the group consisting of cyclopentane and dimethylbutane.

Preferably, the medication infusion pump comprises a plurality of gases within the reservoir, wherein the plurality of gases has a combined vapor pressure less than 14.7 psia at 37°C.

Preferably, the medication chamber and the reservoir are separated by a barrier which is resistant to the gas.

In a preferred embodiment, a method of supplying a medication to a patient from a medication infusion pump, the infusion pump including a housing, a medication chamber defined within the housing, a pumping mechanism for supplying the medication from the medication chamber to the patient, and a reservoir defined within the housing and adapted to apply a predetermined negative pressure to a medication within the medication chamber, comprises the steps of
(i) filling the reservoir with a gas having a vapor pressure less than ambient atmospheric pressure at normal human physiological temperature,
(ii) drawing the medication into the medication chamber by exertion of the predetermined negative pressure on the medication, and
(iii) dispensing the medication from the medication chamber via the pumping mechanism.

Preferably, the gas comprises a non-ozone depleting gas.

Preferably, the gas comprises a non-toxic gas. In a preferred embodiment, there is included a non-toxic and non-ozone depleting gas.

Preferably, the gas has a vapor pressure less than 14.7 psia at 37°C.

Preferably, the gas has a vapor pressure less than 10.7 psia at 37°C.

Preferably, the gas is selected from the group consisting of cyclopentane, 2,2-dimethylbutane, I ,3-pentadiene and 2-methyl-2-butene.

Preferably, the gas is selected from the group consisting of cyclopentane and 2,2-dimethylbutane.

Preferably, the medication pump is implanted within the body of the patient.

Preferably, there is provided a medication infusion pump adapted to be passively refilled with a medication wherein the medication infusion pump includes a reservoir that contains a gas having a vapor pressure less than ambient atmospheric pressure at normal human physiological temperature.

## Claims

1. A medication infusion pump (10; 110) comprising
a) a housing (12;112),
b) a medication chamber (14;114) defined within the housing (12;112),
c) means for supplying (16;116) the medication from the medication chamber (14;114) to a patient,
d) a reservoir (28;122) defined within the housing (12;112) and adapted to apply a predetermined pressure less than ambient atmospheric pressure to the medication chamber (14;114), and
e) a gas within the reservoir (28;122), the gas having a vapor pressure less than ambient atmospheric pressure at normal human physiological temperature, **characterised in that** the gas is selected from the group consisting of cyclopentane, 2,2-dimethylbutane, 1,3-pentadiene, and 2-methyl-2-butene and does not have an adverse effect on ozone.

2. The medication infusion pump of claim 1, wherein the medication infusion pump is adapted to be passively refilled with a medication.

3. The medication infusion pump of claim 1 wherein the gas comprises a non-toxic gas.

4. The medication infusion pump of claim 1 wherein the gas has a vapor pressure less than 14.7 psia (101 kPa) at 37°C.

5. The medication infusion pump of claim 4 wherein the gas has a vapor pressure less than 10.7 psia (74 kPa) at 37°C.

6. The medication infusion pump of claim 1 wherein the gas is selected from the group consisting of cyclopentane and dimethylbutane.

7. The medication infusion pump of claim 1 comprising a plurality of gases within the reservoir (28;122), wherein the plurality of gases has a combined vapor pressure less than 14.7 psia (101 kPa) at 37°C.

8. The medication infusion pump of claim 1 wherein the medication chamber (14;114) and the reservoir (28;122) are separated by a barrier (124) which is resistant to gas.

9. A medication infusion pump for use in a method of supplying a medication to a patient from a medication infusion pump, the infusion pump including a housing (12;112), a medication chamber (14;114) defined within the housing (12;112), a pumping mechanism (16;116) for supplying the medication from the medication chamber (14;114) to the patient, and a reservoir (28;122) defined within the housing (12;112) and adapted to apply a predetermined pressure less than ambient atmospheric pressure to the medication chamber (14;114), the method comprising the steps of
(i) filling the reservoir with a gas having a vapor pressure less than ambient atmospheric pressure at normal human physiological temperature, the gas being selected from the group consisting of cyclopentane, 2,2-dimethylbutane, 1,3-pentadiene, and 2-methyl-2-butene, and wherein the gas does not have an adverse effect on ozone,
ii) drawing the medication into the medication chamber by exertion of the predetermined negative pressure on the medication, and
iii) dispensing the medication from the medication chamber via the pumping mechanism.
